(19) Europäisches Patentamt
European Patent Office
Office européen des brevets

(11) EP 2 538 944 B1

(12) **EUROPEAN PATENT SPECIFICATION**

(45) Date of publication and mention of the grant of the patent:
**22.10.2014 Bulletin 2014/43**

(21) Application number: **11748101.0**

(22) Date of filing: **25.02.2011**

(51) Int Cl.:
***A61K 31/47*** (2006.01) ***A61K 33/24*** (2006.01)
***A61P 35/00*** (2006.01) ***A61K 31/495*** (2006.01)
***A61K 31/555*** (2006.01)

(86) International application number:
**PCT/US2011/026144**

(87) International publication number:
**WO 2011/106577 (01.09.2011 Gazette 2011/35)**

(54) **COMPOUND FOR TREATING BRAIN CANCER**

VERBINDUNG ZUR BEHANDLUNG VON GEHIRNTUMOREN

COMPOSÉ POUR LE TRAITEMENT DU CANCER DU CERVEAU

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **17.11.2010 US 414882 P**
**26.02.2010 US 308813 P**

(43) Date of publication of application:
**02.01.2013 Bulletin 2013/01**

(73) Proprietor: **Niiki Pharma Inc.**
**Hoboken, New Jersey 07030 (US)**

(72) Inventors:
- **SHESHBARADARAN, Hooshmand**
**Hoboken, New Jersey 07030 (US)**
- **BAERGA, Rebecca**
**Hoboken, New Jersey 07030 (US)**
- **COBB, Jenel**
**Hoboken, New Jersey 07030 (US)**
- **VALIAHDI, Seied Mojtaba**
**A-1090 Vienna (AT)**
- **KEPPLER, Bernhard**
**A-1090 Vienna (AT)**

(74) Representative: **Vossius & Partner**
**Siebertstrasse 4**
**81675 München (DE)**

(56) References cited:
**WO-A2-2011/133479 US-A1- 2002 128 228**

- **SEIED MOJTABA VALIAHDI ET AL: "The gallium complex KP46 exerts strong activity against primary explanted melanoma cells and induces apoptosis in melanoma cell lines", MELANOMA RESEARCH, vol. 19, no. 5, 1 October 2009 (2009-10-01), pages 283-293, XP055071628, ISSN: 0960-8931, DOI: 10.1097/CMR. 0b013e32832b272d**
- **TIMERBAEV, A.R.: 'Advances in developing tris (8-quinolinolato)gallium(III) as an anticancer drug: critical appraisal and prospects' METALLOMICS vol. 1, 2009, pages 193 - 198, XP055070390**
- **HEFFETER, P. ET AL.: 'Resistance against novel anticancer metal compounds: Differences and similarities' DRUG RESISTANCE UPDATES vol. 11, 2008, pages 1 - 16, XP022624656**
- **ALAMA, A. ET AL.: 'Organometallic compounds in oncology: implications of novel organotins as antitumor agents' DRUG DISCOVERY TODAY vol. 14, no. 9/10, 2009, pages 500 - 508, XP026085413**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 538 944 B1

## Description

Cross-Reference to Related U.S. Applications

This application claims the benefit of U.S. Provisional Application No. 61/308,813 filed on February 26, 2010 and U.S. Provisional Application No. 61/414,882 filed on November 17, 2010.

Field of the Invention

The present invention generally relates to pharmaceutical compositions and compounds for use in treating cancer, and particularly to a pharmaceutical composition having tris(8-quinolinolato) gallium(III), as defined in the claims.

Background of the Invention

It is estimated that there are over 40,000 new cases of brain cancer every year in the United States alone, and more than 13,000 die each year from the disease. Aside from surgery and radiation therapy, there are very few treatment options. Temozolomide and nitrosourea are the only accepted chemotherapeutics for brain cancer, and yet have shown rather limited effectiveness. Thus, there is a significant need for new agents in treating brain cancer.

US Patent Publication No. 2009/0137620 discloses that the compound tris(8-quinolinolato)gallium(III) has been shown to be effective in causing apoptosis and cell death in melanoma cell lines. However, it is unknown whether the compound is useful in treating brain cancer, especially those refractory to other anti-cancer drugs.

Seied Mojtaba Valiahdi et AL, MELANOMA RESEARCH, vol. 19, no. 5, 1 October 2009, pages 283 - 293 discloses that tris(8-quinolinolato)gallium(III) (KP46) is effective to induce. Cell death in glioblastoma cell lines of astrocytic origin such as T98G and U373 cell lines.

Summary of the Invention

The present invention provides the following embodiments:

1. A compound of Formula (I)

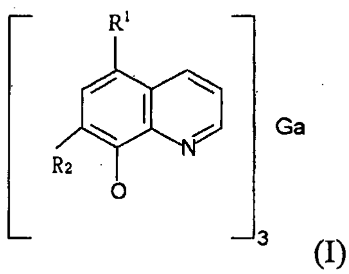

(I)

wherein $R^1$ represents hydrogen, a halogen or a sulfono group $SO_3M$, in which M is a metal ion, and $R^2$ represents hydrogen, or $R^1$ is Cl and $R^2$ is I, or a pharmaceutically acceptable salt thereof for use in the treatment of brain cancer, wherein said brain cancer is a refractory brain cancer, and further wherein said brain cancer was previously treated with BCNU.

2. Use of a compound as defined in embodiment 1 or a pharmaceutically acceptable salt thereof for the manufacture of a medicament for treating brain cancer, wherein said brain cancer is a refractory brain cancer, and further wherein said brain cancer was previously treated with BCNU.

3. A compound of Formula (I)

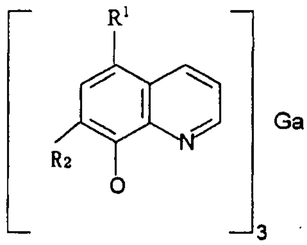

(I)

wherein $R^1$ represents hydrogen, a halogen or a sulfono group $SO_3M$, in which M is a metal ion, and $R^2$ represents hydrogen, or $R^1$ is Cl and $R^2$ is I, or a pharmaceutically acceptable salt thereof for use in the treatment of brain cancer, wherein said compound is to be used in combination with temozolomide.

4. Use of a compound as defined in embodiment 3 or a pharmaceutically acceptable salt thereof for the manufacture of a medicament for treating brain cancer, wherein said compound is to be used in combination with temozolomide.

5. The compound for use of embodiment 1 or 3 or the use of embodiment 2 or 4, wherein said compound is tris(8-quinolinolato)gallium(III).

6. The compound for use of any one of embodiments 1, 3 and 5 or the use of any one of embodiments 2, 4 and 5, wherein said brain cancer is not glioblastoma.

7. The compound for use of any one of embodiments 1, 3, 5 and 6 or the use of any one of embodiments 2 and 4 to 6, wherein said brain cancer is astrocytoma.

8. The compound for use of any one of embodiments 1, 3 and 5 or the use of any one of embodiments 2, 4 and 5, wherein said brain cancer is glioblastoma.

9. The compound for use of any one of embodiments 3 to 8 or the use of any one of embodiments 4 to 8, wherein said brain cancer is a refractory brain cancer.

10. The compound for use of embodiment 9 or the use of embodiment 9, wherein said brain cancer was previously treated with BCNU.

11. The compound for use of embodiment 9 or the use of embodiment 9, wherein said brain cancer was previously treated with temozolomide.

12. A kit comprising:

an effective amount of a compound of Formula (I)

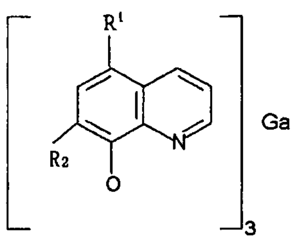

(I)

wherein $R^1$ represents hydrogen, a halogen or a sulfono group $SO_3M$, in which M is a metal ion, and $R^2$ represents hydrogen, or $R^1$ is Cl and $R^2$ is I, or a pharmaceutically acceptable salt thereof; and an effective amount of temozolomide.

13. The kit of embodiment 12, wherein said compound is tris(8-quinolinolato)gallium(III).

14. A pharmaceutical composition comprising: an effective amount of a compound of Formula (I)

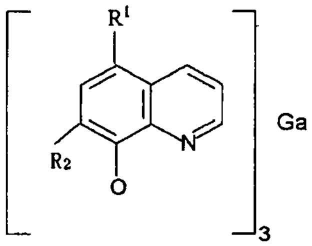

(I)

wherein $R^1$ represents hydrogen, a halogen or a sulfono group $SO_3M$, in which M is a metal ion, and $R^2$ represents hydrogen, or $R^1$ is Cl and $R^2$ is I, or a pharmaceutically acceptable salt thereof; and an effective amount of temozolomide.

15. The pharmaceutical composition of embodiment 14, wherein said compound is tris(8-quinolinolato)gallium(III).

In one aspect, the present invention provides a compound according to Formula (I) below or a pharmaceutically acceptable salt thereof (e.g., tris(8-quinolinolato)gallium(III)) for use in treating a refractory brain cancer, comprising administering a therapeutically effective amount of the compound according to Formula (I) or a pharmaceutically acceptable salt thereof (e.g., tris(8-quinolinolato)gallium(III)) to a patient refractory to a treatment comprising at least BCNU and optionally temeozolomide.

In another aspect, a compound according to Formula (I) below or a pharmaceutically acceptable salt thereof (e.g., tris(8-quinolinolato)gallium(III)) for use in treating brain cancer is provided, comprising administering to a cancer patient in need of treatment, simultaneously or sequentially, a therapeutically effective amount of (1) the compound according to Formula (I) or a pharmaceutically acceptable salt thereof (e.g., tris(8-quinolinolato)gallium(III)), and (2) temozolomide. In specific embodiments, the combination is used for the treatment of glioblastoma or astrocytoma.

Use of the compound according to Formula (I) below or a pharmaceutically acceptable salt thereof (e.g., tris(8-quinolinolato)gallium(III)) for the manufacture of a medicament for treating brain cancer as defined in the claims is also provided.

The foregoing and other advantages and features of the invention, and the manner in which the same are accomplished, will become more readily apparent upon consideration of the following detailed description of the invention taken in conjunction with the accompanying examples, which illustrate preferred and exemplary embodiments.

## Brief Description of Drawings

**Figure 1** is a graph showing the dose-dependent growth inhibition by tris(8-quinolinolato)gallium(III) (MTT assay) in a 3-dimensional tumor model (HuBiogel, Vivo Biosciences, Birmingham, AL) derived from glioma cell line U87. X axis is drug concentration in μM and Y axis is percentage of control;

**Figure 2** is a combination index plot illustrating the additive to synergistic activity between tris(8-quinolinolato)gallium(III) and temozolomide in the glioblastoma cell line U251.

## Detailed Description of the Invention

The present invention is at least in part based on the discovery that the compound tris(8-quinolinolato)gallium(III) is especially effective in treating brain cancers. Accordingly, in accordance with a first aspect of the present invention, a compound according to Formula (I) below or a pharmaceutically acceptable salt thereof (e.g., tris(8-quinolinolato)gallium(III)) for use in treating brain cancer is provided, as further defined in the claims. The use comprises treating a brain cancer patient in need of treatment with a therapeutically effective amount of a gallium complex of Formula (I)

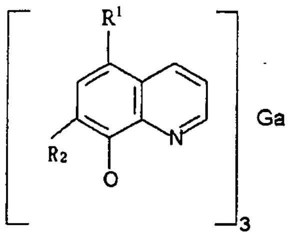

(I)

wherein $R^1$ represents hydrogen, a halogen or a sulfono group $SO_3M$, in which M is a metal ion, and $R^2$ represents hydrogen, or $R^1$ is Cl and $R^2$ is I, or a pharmaceutically acceptable salt thereof as defined in the claims. Brain tumor is an intracranial solid neoplasm within the brain or the central spinal canal. In one embodiment,
the brain cancer is not glioblastoma. That is, the present invention is directed to the use of an effective amount of a compound according to Formula (I) or a pharmaceutically acceptable salt thereof for the manufacture of medicaments for treating a brain cancer in patients identified or diagnosed as having a brain cancer, optionally said brain cancer not being glioblastoma, as defined in the claims.

In the various embodiments of this aspect of the present invention, the treatment optionally also comprises a step of diagnosing or identifying a patient as having brain tumor. The identified patient is then treated with or administered with a therapeutically effective amount of a compound of the present invention, e.g., tris(8-quinolinolato)gallium(III) which has a formula:

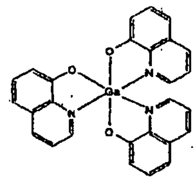

Various brain cancers can be diagnosed in any conventional diagnostic methods known in the art including MRI scan, CAT scan, PET scan, biopsy, etc.

In addition, it has also been surprisingly discovered that the compound tris(8-quinolinolato)gallium(III) is equally effective in brain cancer cells resistant to nitrosourea (e.g., bis-chloronitrosourea (BCNU)) or temozolomide. Accordingly, another aspect of the present invention provides a compound for use in treating refractory brain cancer comprising treating a patient identified as having refractory brain cancer with a therapeutically effective amount of a compound of Formula (I) or a pharmaceutically acceptable salt thereof (e.g., tris(8-quinolinolato)gallium(III)), wherein the patient has a brain cancer that is refractory to a treatment comprising BCNU

The term "refractory brain cancer," as used herein refers to a brain cancer that either fails to respond favorably to an anti-neoplastic treatment that does not include a compound of Formula (I), or alternatively, recurs or relapses after responding favorably to an antineoplastic treatment that does not include a compound of Formula (I). Accordingly, "a brain cancer refractory to a treatment" as used herein means a brain cancer that fails to respond favorably to, or resistant to, the treatment, or alternatively, recurs or relapses after responding favorably to the treatment.

Thus, in some embodiments of the present invention, a compound of Formula (I) or a pharmaceutically acceptable salt thereof (e.g., tris(8-quinolinolato)gallium(III)) is used to treat brain cancer patients having a tumor previously treated with a treatment regimen comprising one or more drugs such as nitrosourea (e.g., BCNU) and temozolomide, as defined in the claims. For example, the compound is used to treat a brain cancer patient having previously been treated with a treatment regimen that includes one or more drugs such as BCNU and temozolomide, and whose brain cancer was found to be non-responsive to the treatment regimen or have developed resistance to the treatment regimen. In other embodiments, the compound is used to treat a brain cancer patient previously treated with a treatment comprising one or more drugs such as BCNU and temozolomide, but the brain cancer has recurred or relapsed, that is, a brain cancer patient who has previously been treated with one or more such drugs, and whose cancer was initially responsive to the previously administered one or more such drugs, but was subsequently found to have relapsed.

In specific embodiments, a compound of Formula (I) or a pharmaceutically acceptable salt thereof (e.g., tris(8-quinolinolato)gallium(III)) is used to treat brain cancer patients previously treated with BCNU.

In yet other specific embodiments, a compound of Formula (I) or a pharmaceutically acceptable salt thereof (e.g., tris(8-quinolinolato)gallium(III)) is used to treat brain cancer patients previously treated with temozolomide, i.e., who have a brain cancer that exhibits resistance to, or relapsed after, a treatment including, temozolomide, as defined in the claims.

To detect a refractory brain cancer, patients undergoing initial treatment can be carefully monitored for signs of resistance, non-responsiveness or recurring brain cancer. This can be accomplished by monitoring the patient's cancer's response to the initial treatment which, e.g., may include nitrosourea or temozolomide. The response, lack of response, or relapse of the cancer to the initial treatment can be determined by any suitable method practiced in the art. For example, this can be accomplished by the assessment of tumor size and number. An increase in tumor size or, alternatively, tumor number, indicates that the tumor is not responding to the chemotherapy, or that a relapse has occurred. The determination can be done according to the "RECIST" criteria as described in detail in Therasse et al, J. Natl. Cancer Inst. 92:205-216 (2000).

In addition, it has been surprisingly discovered that the combination of tris(8-quinolinolato)gallium(III) and temozolomide provided a significant synergy in causing apoptosis in glioblastoma cells. Thus, the present invention also provides a combination therapy, in which temozolomide and the compound of Formula (I) (e.g., tris(8-quinolinolato)gallium(III)) are used together in the same treatment regimen. That is, the present invention provides a compound of Formula (I) or a pharmaceutically acceptable salt thereof for use in treating brain cancer, comprising administering to a brain cancer patient in need of treatment, simultaneously or sequentially, a therapeutically effective amount of (1) the compound according to Formula (I) or a pharmaceutically acceptable salt thereof (e.g., tris(8-quinolinolato)gallium(III)), and (2) temozolomide. An effective amount of additional drugs such as bevacizumab, nitrosourea and procarbazine may also be optionally included in the combination therapy and administered to the brain cancer patient. In specific embodiments, the combination is used for the treatment of glioblastoma multiforme (GBM) or anaplastic astrocytoma. In preferred embodiments, the gallium complex is tris(8-quinolinolato)gallium(III) or a pharmaceutically acceptable salt thereof

The various aspects of the present invention can be useful in various brain malignancies including, acoustic neuroma, astrocytoma (e.g., pilocytic astrocytoma, low-grade astrocytoma, anaplastic astrocytoma), glioblastoma multiforme (GBM) and other gliomas (brain stem glioma, optic nerve glioma, ependymoma, mixed glioma, optic nerve glioma, oligodendroglioma, and subependymoma), chordoma, CNS lymphoma, craniopharyngioma, medulloblastoma, meningioma, pituitary tumors, primitive neuroectodermal (PNET), schwannoma, pineal tumor and rhabdoid tumor.

As used herein, the phrase "treating . . . with . . ." or a paraphrase thereof means administering a compound to the patient or causing the formation of a compound inside the body of the patient.

In accordance with the present invention and as defined in the claims, brain cancer can be treated with a therapeutically effective amount of a compound of Formula (I) or a pharmaceutically acceptable salt thereof (e.g., tris(8-quinolinolato)gallium(III)) alone as a single agent, or alternatively in combination with one or more other anti-cancer agents.

The pharmaceutical compounds of Formula (I) can be administered through intravenous injection or oral administration or any other suitable means at an amount of from 0.1 mg to 1000 mg per kg of body weight of the patient based on total body weight. The active ingredients may be administered at predetermined intervals of time, e.g., three times a day. It should be understood that the dosage ranges set forth above are exemplary only and are not intended to limit the scope of this invention. The therapeutically effective amount of the active compound can vary with factors including, but not limited to, the activity of the compound used, stability of the active compound in the patient's body, the severity of the conditions to be alleviated, the total weight of the patient treated, the route of administration, the ease of absorption, distribution, and excretion of the active compound by the body, the age and sensitivity of the patient to be treated, and the like, as will be apparent to a skilled artisan. The amount of administration can be adjusted as the various factors change over time.

In accordance with the present invention, it is provided a use of a compound of Formula (I) or a pharmaceutically acceptable salt thereof (e.g., tris(8-quinolinolato)gallium(III)) for the manufacture of a medicament useful for treating brain cancer alone or in combination of temozolomide, as defined in the claims. The medicament can be, e.g., in an oral or injectible form, e.g., suitable for intravenous, intradermal, or intramuscular administration. Injectable forms are generally known in the art, e.g., in buffered solution or suspension.

In accordance with another aspect of the present invention, a pharmaceutical kit is provided comprising in a container a unit dosage form of a compound of Formula (I) or a pharmaceutically acceptable salt thereof (e.g., tris(8-quinolinolato)gallium(III)), and optionally instructions for using the invention, e.g., treating brain cancer, or treating refractory brain cancer, as defined in the claims. In one embodiment, a pharmaceutical kit is provided comprising in a compartmentalized container (1) a unit dosage form of a compound of Formula (I) or a pharmaceutically acceptable salt thereof (e.g., tris(8-quinolinolato)gallium(III)); and (2) a unit dosage form of temozolomide. As will be apparent to a skilled artisan, the amount of a therapeutic compound in the unit dosage form is determined by the dosage to be used on a patient in accordance with the present invention. In the kit, a compound of Formula (I) or a pharmaceutically. acceptable salt thereof (e.g., tris(8-quinolinolato)gallium(III)) can be in a tablet form in an amount of, e.g., 1 mg. Temozolomide to be used in the combination therapy and included in the kit can be in any dosage form generally known or used in the art, e.g., tablet, capsule, a lyophilized form for reconstitution of an injectable form, etc. Optionally, the kit further comprises instructions for using the kit in the combination therapy in accordance with the present invention.

In accordance with another aspect of the present invention, a pharmaceutical composition is provided, comprising an effective amount of (1) a compound of Formula (I) or a pharmaceutically acceptable salt thereof (e.g., tris(8-quinolinolato)gallium(III)); and (2) temozolomide. The pharmaceutical composition can be in any pharmaceutically acceptable dosage forms including tablet, capsule, a lyophilized form for reconstitution of an injectable form, solution or suspension, etc. The amount of the drugs to be included in the composition can vary and depend on the amount to be administered to a patient. For example, the compound of Formula (I) or a pharmaceutically acceptable salt thereof (e.g., tris(8-quinolinolato)gallium(III)) can be included in the composition at an amount of from 1 mg to about 1000 mg, and temozolomide can be at 5 mg, 20 mg, 100 mg, 140 mg, 180 mg or 250 mg

EXAMPLE 1

To test the activities of tris(8-quinolinolato)gallium(III) ("drug"), MTT assays were performed using glioblastoma cell lines. Cells were plated ($2x10^3$ cells in 100 μl/well) in 96-well plates and allowed to recover for 24 hours. The drug was added in another 100 μl growth medium and incubated with cultured cells for 3 hours before the cell culture medium was replaced to remove the drug. Cell death was measured 72 hours after the initial incubation by MTT assay following the manufacturer's recommendations (EZ4U, Biomedica, Vienna, Austria). The cell lines tested and $IC_{50}$ values (drug concentration with 50% growth inhibition) are summarized below in Table 1. Tris(8-quinolinolato)gallium(III) was effective in inducing cell death in all cell lines in Table 1 with $IC_{50}$ values ranging from about 0.3 μM to about 2.5 μM. Historical data on the inhibition of the cell lines by the nitrosourea drug BCNU are provided in Table 1 below. It is noted that tris(8-quinolinolato)gallium(III) is active against U87 cell line which is relatively resistant to BCNU. In addition, tris(8-quinolinolato)gallium(III) is active in T98G cells which have been shown to be resistant to temozolomide. *See* Kanzawa et al., J. Neurosurg., 99:1047-1052 (2003).

Table 1

| Cell Line | Drug $IC_{50}$ (μm) | BCNU (% cytostasis with 50 μg/ml BCNU)* |
|---|---|---|
| ACHU | 0.3 | |
| T98G | 2.0 | 37.5±8.6 |
| U87 | 0.94 | 18.5±8.2 |
| U373 | 2.5 | 30.4±8.6 |
| * historical data from Bowles et al., J. Neurosurg., 73:248-253 (1990). | | |

EXAMPLE 2

The compound tris(8-quinolinolato)gallium(III) was tested in a 3-dimentional tumor model derived from glioma cell line U87. Specifically, cells were trypsinized, washed, counted by trypan blue exclusion. Tumor beads were then prepared by mixing 20,000 cells/10μl ofHuBiogel (4 mg/mL) *(See* US Patent Application Serial No. 10/546,506, which is incorporated herein by reference). The 3-D tumor beads were cultivated for 72 hours in multi-well plates with complete media (10% FBS) in a 37°C incubator +5% $CO_2$. Mini-tumors were treated with various concentrations of the test compound tris(8-quinolinolato)gallium(III) in media (final 0.2-0.3% DMSO) or control (DMSO). Repeated drug treatment was done by removing the culture media and replacing with fresh media with drug compound or DMSO. On Day 3, MTT assay and live-cell staining with Calcein AM were performed (5 beads/assay set).

Tris(8-quinolinolato)gallium(III) exhibited dose-dependent tumor killing effective in live-cell staining/image analysis, and significantly inhibited tumor proliferation activity. *See* Figure 1. Statistical analysis of data sets (Average, T-test, GI-50) was performed using MS-Excel program. The T-test result is shown in Table 2 below. The average GI-50 (the drug concentration required for growth inhibition at 50%) is 0.94 μM.

Table 2

| | **Concentration** (μM) | | | |
|---|---|---|---|---|
| **t-test** | **8** | **4** | **2** | **1** |
| HuBiogel (control vs experiment) | 1.12672E-11 | 7.18966E-10 | 3.82139E-11 | 3.60697E-06 |
| | control vs 8 μM | control vs 4 μM | control vs 2 μM | control vs 1 μM |

EXAMPLE 3

**Activities of Tris(8-quinolinolato)gallium(III) in Human Glioblastoma Cell Line Resistant to Temozolomide and BCNU**

Tris(8-quinolinolato)gallium(III) and temozolomide were tested in human glioblastoma cell line U251 (which is resistant to BCNU, *See* Beljanski et al., Anticancer Res., 13(6A):2301-8 (1993)). Specifically, ATCC's MTT Cell Proliferation Assay® was performed using glioblastoma cell line U251. Stock cultures were allowed to grow to 70-80% confluence for this study. The anti-proliferative activity of tris(8-quinolinolato)gallium(III) or temozolomide against the cell line was evaluated *in vitro* using the ATCC's MTT Cell Proliferation Assay (Catalog No. 30-1010K). Cultures were maintained in a 37°C humidified 5% $CO_2$/95% air atmosphere. The cells were treated with tris(8-quinolinolato)gallium(III) or temozolomide at 1,000 μM, or a series of 4x dilutions thereof (250 μM, 62.5 μM, etc.). 100μl of medium was removed from each well at 72 hours post-treatment and 10μl MTT reagent was added to each well. The plates were incubated at 37°C for 4 hours and then 100 μl of detergent was added. The plates were left overnight at room temperature in the dark and was read on a plate reader using SoftMax® Pro (version 5.2, Molecular Devices).

The absorbance data was analyzed as follows: Absorbance values were converted to Percent of Control and plotted against test agent concentrations for $IC_{50}$ calculations using SoftMax® Pro (version 5.2, Molecular Devices). The plate blank signal average was subtracted from all wells prior to calculating the Percent of Control. Percent of Control values were calculated by dividing the absorbance values for each test well by the No Drug Control average (column 11 values; cells + vehicle control) and multiplying by 100. Plots of Compound Concentration versus Percent of Control were analyzed using the 4-parameter equation to obtain $IC_{50}$ values and other parameters that describe the sigmoidal dose response curve.

The $IC_{50}$ value for the test agent was estimated by curve-fitting the data using the following four parameter-logistic equation:

$$Y = \frac{Top - Bottom}{1 + \left( X / IC_{50} \right)^n} + Bottom$$

wherein "Top" is the maximal % of control absorbance (100%), "Bottom" is the minimal % of control absorbance at the highest agent concentration (down to zero), Y is the Percent of Control absorbance, X is the test agent Concentration, $IC_{50}$ is the concentration of agent that inhibits cell growth by 50% compared to the control cells, n is the slope of the curve.

The $IC_{50}$ of tris(8-quinolinolato)gallium(III) in the U251 cell line was 4.04 μM whereas the $IC_{50}$ of temozolomide was 283 μM and that of BCNU was 53.7 μM. Thus, tris(8-quinolinolato)gallium(III) is effective in glioblastoma cells resistant to temozolomide or BCNU

**EXAMPLE 4**

**Combination Studies**

Human glioblastoma cell line U251 cells were cultured under conditions described in Example 3. The cells were sub-cultured regularly to maintain log phase growth. On the day of $EC_{50}$ plate seeding, the cells were processed and seeded into 96-well cell culture-treated plates one cell line at a time. The cells were removed from their culture flasks using trypsin solution pooled in a sterile conical tube and centrifuged at 350xg for 5 minutes at room temperature. Pelleted cells were re-suspended in complete media and then counted with a Neubauer Bright-Line® hemacytometer and trypan blue viability stain. The cell suspensions were diluted (based on live cell counts) using complete media to yield a final suspension density (cells/ml) based on previously determined seeding densities for each cell line for a 72 hour 96-well plate assay. The tissue culture treated plates for $EC_{50}$ testing were seeded at 1.5 x $10^3$ cells/well, and incubated overnight at 37°C in a 5% $CO_2$, 95% air humidified atmosphere to allow the cells to attach.

**Test Agent Preparation:** For each single agent or combination of test agents, the top concentration mixture (2x final treatment concentration) was made in sterile 1.5 ml microcentrifuge tubes and then directly transferred to the first well of the treatment dilution plates.

Tris(8-quinolinolato)gallium(III) was obtained from Niiki Pharma, Inc. Temozolomide was manufactured by the Schering Corporation and supplied in an amber glass vial. It was stored in the dark at room temperature and sealed with Parafilm® to limit exposure to light and humidity. Temozolomide (20.7 mg) was weighed out and a 400 mM white, cloudy suspension was made by adding 149μL of 100% DMSO and brief sonication (~10-20seconds) in a sonicating

water bath without heat.

The antiproliferative activity of the test agents was evaluated using the MTT Cell Proliferation Assay Kit (ATCC catalog # 30-1010K). Cells in the log phase of growth were seeded at the indicated density into 96-well culture treated plates in 0.1 mL of complete media in all wells except for one column reserved for the media only control. The cells were allowed to attach during an overnight incubation prior to treating with test agents. Test agents were serially diluted in complete culture media (+1% DMSO where appropriate) and added to each well in a volume of 0.1 mL for a total final volume of 0.2 mL/well (0.5% DMSO final, where used). Cells were exposed to test agents for 72 hours. Following the exposure to test agents, 0.1 mL of culture supernatant was carefully removed from all wells of each plate and 0.01 mL of MTT reagent was added to each well. The plates were returned to the incubator for four hours. Following the incubation period, kit supplied detergent reagent (0.1 mL) was added to all wells. The plates were wrapped in plastic wrap to prevent evaporation and allowed to sit at room temperature in the dark overnight. The absorbance at 570 nm was measured the following day using a SpectraMAX Plus plate reader (Molecular Devices). Absorbance values were converted to Percent of Control and plotted against test agent concentrations for $EC_{50}$ calculations using SoftMax® Pro (version 5.2, Molecular Devices). The plate blank signal average was subtracted from all wells prior to calculating the Percent of Control. Percent of Control values were calculated by dividing the absorbance values for each test well by the No Drug Control average (column 11 values; cells + vehicle control) and multiplying by 100. Plots of Compound Concentration vs. Percent of Control were analyzed using the 4-parameter equation to obtain $EC_{50}$ values and other parameters that describe the sigmoidal dose response curve.

Combination data was analyzed using CompuSyn® software to calculate Combination Index (CI) values to assess synergy. The Fractional Affect (Fa) was calculated from the Pecent of Control (from SoftMax® Pro) using the formula: 1-(Percent Control/100). The dosage, fractional affect and molar ratio of compounds tested in combination were entered into the CompuSyn® software for evaluation of the presence/absence of synergy. CompuSyn® assigns a Combination Index (CI) value which rates the level of compounds' affect on proliferation. CI values below 1 indicate the presence of synergy and CI values above 1 indicate antagonism. CI values close to 1 indicate an additive affect. *See* Chou, Pharmacol. Rev., 58(3):621-81 (2006). The combination of tris(8-quinolinolato)gallium(III) and temozolomide had a CI value of 0.812 in U251 cells, indicating a synergistic combination. **Figure 2** is a combination index plot illustrating the additive to synergistic activity between tris(8-quinolinolato)gallium(III) and temozolomide in the glioblastoma cell line U251.

## Claims

1. A compound of Formula (I)

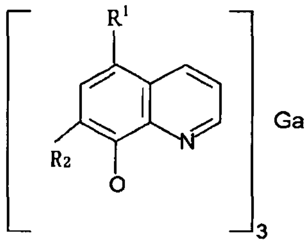

(I)

wherein $R^1$ represents hydrogen, a halogen or a sulfono group $SO_3M$, in which M is a metal ion, and $R^2$ represents hydrogen, or $R^1$ is Cl and $R^2$ is I, or a pharmaceutically acceptable salt thereof for use in the treatment of brain cancer, wherein said brain cancer is a refractory brain cancer, and further wherein said brain cancer was previously treated with BCNU (bis-chloronitrosourea).

2. Use of a compound as defined in Claim 1 or a pharmaceutically acceptable salt thereof for the manufacture of a medicament for treating brain cancer, wherein said brain cancer is a refractory brain cancer, and further wherein said brain cancer was previously treated with BCNU (bis - chloronitrosourea).

3. A compound of Formula (I)

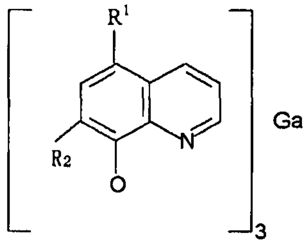

(I)

wherein $R^1$ represents hydrogen, a halogen or a sulfono group $SO_3M$, in which M is a metal ion, and $R^2$ represents hydrogen, or $R^1$ is Cl and $R^2$ is I, or a pharmaceutically acceptable salt thereof for use in the treatment of brain cancer, wherein said compound is to be used in combination with temozolomide.

**4.** Use of a compound as defined in Claim 3 or a pharmaceutically acceptable salt thereof for the manufacture of a medicament for treating brain cancer, wherein said compound is to be used in combination with temozolomide.

**5.** The compound for use of Claim 1 or 3 or the use of Claim 2 or 4, wherein said compound is tris(8-quinolinolato)gallium(III).

**6.** The compound for use of any one of Claims 1, 3 and 5 or the use of any one of Claims 2, 4 and 5, wherein said brain cancer is not glioblastoma.

**7.** The compound for use of any one of Claims 1, 3, 5 and 6 or the use of any one of Claims 2 and 4 to 6, wherein said brain cancer is astrocytoma.

**8.** The compound for use of any one of Claims 1, 3 and 5 or the use of any one of Claims 2, 4 and 5, wherein said brain cancer is glioblastoma.

**9.** The compound for use of any one of Claims 3 to 8 or the use of any one of Claims 4 to 8, wherein said brain cancer is a refractory brain cancer.

**10.** The compound for use of Claim 9 or the use of Claim 9, wherein said brain cancer was previously treated with BCNU.

**11.** The compound for use of Claim 9 or the use of Claim 9, wherein said brain cancer was previously treated with temozolomide.

**12.** A kit comprising:

an effective amount of a compound of Formula (I)

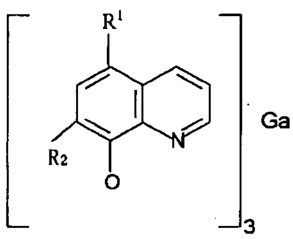

(I)

wherein $R^1$ represents hydrogen, a halogen or a sulfono group $SO_3M$, in which M is a metal ion, and $R^2$ represents hydrogen, or $R^1$ is Cl and $R^2$ is I, or a pharmaceutically acceptable salt thereof; and an effective amount of temozolomide.

**13.** The kit of Claim 12, wherein said compound is tris(8-quinolinolato)gallium(III).

**14.** A pharmaceutical composition comprising: an effective amount of a compound of Formula (I)

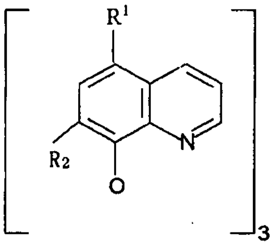

(I)

wherein $R^1$ represents hydrogen, a halogen or a sulfono group $SO_3M$, in which M is a metal ion, and $R^2$ represents hydrogen, or $R^1$ is Cl and $R^2$ is I, or a pharmaceutically acceptable salt thereof; and an effective amount of temozolomide.

**15.** The pharmaceutical composition of Claim 14, wherein said compound is tris(8-quinolinolato)gallium(III).

**Patentansprüche**

**1.** Eine Verbindung der Formel (I)

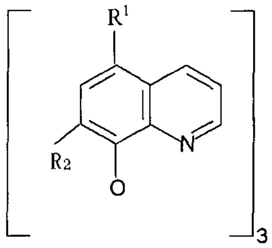

(I)

wobei $R^1$ Wasserstoff, ein Halogenatom oder eine Sulfonogruppe $SO_3M$, in welcher M ein Metallion ist, darstellt und $R^2$ Wasserstoff darstellt, oder $R^1$ für Cl und $R^2$ für I steht, oder ein pharmazeutisch verträgliches Salz davon zur Verwendung bei der Behandlung von Gehirntumor, wobei der Gehirntumor ein refraktärer Gehirntumor ist, und wobei weiterhin der Gehirntumor im Vorfeld mit BCNU (Bis-Chlornitrosoharnstoff) behandelt wurde.

**2.** Verwendung einer Verbindung wie in Anspruch 1 definiert oder eines pharmazeutisch verträglichen Salzes davon zur Herstellung eines Medikaments zur Behandlung von Gehirntumor, wobei der Gehirntumor ein refraktärer Gehirntumor ist, und wobei weiterhin der Gehirntumor im Vorfeld mit BCNU (Bis-Chlornitrosoharnstoff) behandelt wurde.

**3.** Eine Verbindung der Formel (I)

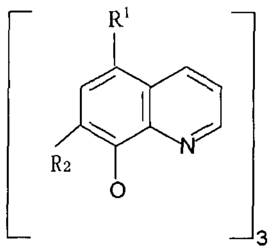

(I)

wobei $R^1$ Wasserstoff, ein Halogenatom oder eine Sulfonogruppe $SO_3M$, in welcher M ein Metallion ist, darstellt und $R^2$ Wasserstoff darstellt, oder $R^1$ für Cl und $R^2$ für I steht, oder ein pharmazeutisch verträgliches Salz davon

zur Verwendung bei der Behandlung von Gehirntumor, wobei die Verbindung in Kombination mit Temozolomid zu verwenden ist.

4. Verwendung einer Verbindung wie in Anspruch 3 definiert oder eines pharmazeutisch verträglichen Salzes davon zur Herstellung eines Medikaments zur Behandlung von Gehirntumor, wobei die Verbindung in Kombination mit Temozolomid zu verwenden ist.

5. Die Verbindung zur Verwendung gemäß Anspruch 1 oder 3 oder die Verwendung gemäß Anspruch 2 oder 4, wobei die Verbindung Tris(8-chinolinolato)gallium(III) ist.

6. Die Verbindung zur Verwendung gemäß einem der Ansprüche 1, 3 und 5 oder die Verwendung gemäß einem der Ansprüche 2, 4 und 5, wobei der Gehirntumor kein Glioblastom ist.

7. Die Verbindung zur Verwendung gemäß einem der Ansprüche 1, 3, 5 und 6 oder die Verwendung gemäß einem der Ansprüche 2 und 4 bis 6, wobei der Gehirntumor ein Astrocytom ist.

8. Die Verbindung zur Verwendung gemäß einem der Ansprüche 1, 3 und 5 oder die Verwendung gemäß einem der Ansprüche 2, 4 und 5, wobei der Gehirntumor ein Glioblastom ist.

9. Die Verbindung zur Verwendung gemäß einem der Ansprüche 3 bis 8 oder die Verwendung gemäß einem der Ansprüche 4 bis 8, wobei der Gehirntumor ein refraktärer Gehirntumor ist.

10. Die Verbindung zur Verwendung gemäß Anspruch 9 oder die Verwendung gemäß Anspruch 9, wobei der Gehirntumor im Vorfeld mit BCNU behandelt wurde.

11. Die Verbindung zur Verwendung gemäß Anspruch 9 oder die Verwendung gemäß Anspruch 9, wobei der Gehirntumor im Vorfeld mit Temozolomid behandelt wurde.

12. Ein Kit, umfassend:

eine wirksame Menge einer Verbindung der Formel (I)

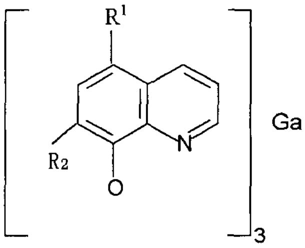

(I)

wobei $R^1$ Wasserstoff, ein Halogenatom oder eine Sulfonogruppe $SO_3M$, in welcher M ein Metallion ist, darstellt und $R^2$ Wasserstoff darstellt, oder $R^1$ für Cl und $R^2$ für I steht,
oder ein pharmazeutisch verträgliches Salz davon; und eine wirksame Menge an Temozolomid.

13. Das Kit gemäß Anspruch 12, wobei die Verbindung Tris(8-chinolinolato)gallium(III) ist.

14. Ein Arzneimittel, umfassend: eine wirksame Menge einer Verbindung der Formel (I)

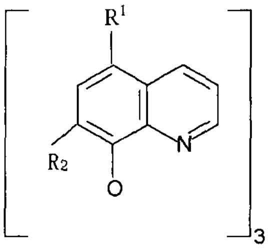

(I)

wobei $R^1$ Wasserstoff, ein Halogenatom oder eine Sulfonogruppe $SO_3M$, in welcher M ein Metallion ist, darstellt und $R^2$ Wasserstoff darstellt, oder $R^1$ für Cl und $R^2$ für I steht, oder ein pharmazeutisch verträgliches Salz davon; und eine wirksame Menge an Temozolomid.

**15.** Das Arzneimittel gemäß Anspruch 14, wobei die Verbindung Tris(8-chinolinolato)gallium(III) ist.

**Revendications**

**1.** Composé de Formule (I)

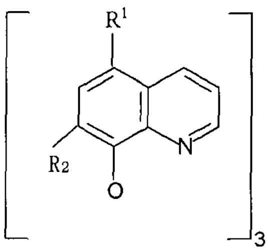

(I)

dans laquelle $R^1$ représente un atome d'hydrogène, un atome d'halogène ou un groupe sulfono $SO_3M$, dans lequel M représente un ion métal, et $R^2$ représente un atome d'hydrogène, ou $R^1$ représente un atome de Cl et $R^2$ représente un atome d'I, ou un sel pharmaceutiquement acceptable de celui-ci pour une utilisation dans le traitement du cancer du cerveau, dans lequel ledit cancer du cerveau est un cancer du cerveau réfractaire, et en outre dans lequel ledit cancer du cerveau a été traité précédemment par de la BCNU (bis-chloronitrosourée).

**2.** Utilisation d'un composé tel que défini dans la revendication 1 ou d'un sel pharmaceutiquement acceptable de celui-ci pour la fabrication d'un médicament destiné à traiter le cancer du cerveau, dans laquelle ledit cancer du cerveau est un cancer du cerveau réfractaire, et en outre dans laquelle ledit cancer du cerveau a été traité précédemment par de la BCNU (bis-chloronitrosourée).

**3.** Composé de Formule (I)

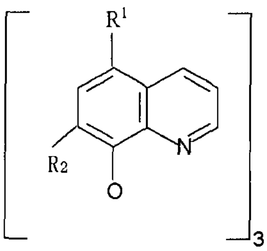

(I)

dans laquelle $R^1$ représente un atome d'hydrogène, un atome d'halogène ou un groupe sulfono $SO_3M$, dans lequel M représente un ion métal, et $R^2$ représente un atome d'hydrogène, ou $R^1$ représente un atome de Cl et $R^2$ représente un atome d'I, ou un sel pharmaceutiquement acceptable de celui-ci pour une utilisation dans le traitement du cancer

du cerveau, dans lequel ledit composé est à utiliser en association avec du témozolomide.

4. Utilisation d'un composé tel que défini dans la revendication 3 ou d'un sel pharmaceutiquement acceptable de celui-ci pour la fabrication d'un médicament destiné à traiter le cancer du cerveau, dans laquelle ledit composé est à utiliser en association avec du témozolomide.

5. Composé pour une utilisation selon la revendication 1 ou la revendication 3 ou une utilisation selon la revendication 2 ou la revendication 4, dans lequel ledit composé est le tris(8-quinolinolato)gallium(III).

6. Composé pour une utilisation selon l'une quelconque des revendications 1, 3 et 5 ou une utilisation selon l'une quelconque des revendications 2, 4 et 5, dans lequel ledit cancer du cerveau n'est pas un glioblastome.

7. Composé pour une utilisation selon l'une quelconque des revendications 1, 3, 5 et 6 ou une utilisation selon l'une quelconque des revendications 2 et 4 à 6, dans lequel ledit cancer du cerveau est un astrocytome.

8. Composé pour une utilisation selon l'une quelconque des revendications 1, 3 et 5 ou une utilisation selon l'une quelconque des revendications 2, 4 et 5, dans lequel ledit cancer du cerveau est un glioblastome.

9. Composé pour une utilisation selon l'une quelconque des revendications 3 à 8 ou une utilisation selon l'une quelconque des revendications 4 à 8, dans lequel ledit cancer du cerveau est un cancer du cerveau réfractaire.

10. Composé pour une utilisation selon la revendication 9 ou utilisation selon la revendication 9, dans lequel ledit cancer du cerveau a été traité précédemment par de la BCNU.

11. Composé pour une utilisation selon la revendication 9 ou utilisation selon la revendication 9, dans lequel ledit cancer du cerveau a été traité précédemment par du témozolomide.

12. Kit comprenant :

une quantité efficace d'un composé de Formule (I)

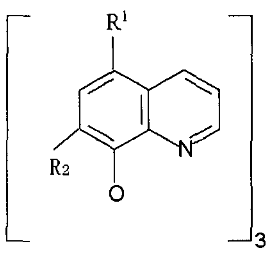

(I)

dans laquelle $R^1$ représente un atome d'hydrogène, un atome d'halogène ou un groupe sulfono $SO_3M$, dans lequel M représente un ion métal, et $R^2$ représente un atome d'hydrogène, ou $R^1$ représente un atome de Cl et $R^2$ représente un atome d'I,
ou d'un sel pharmaceutiquement acceptable de celui-ci ; et une quantité efficace de témozolomide.

13. Kit selon la revendication 12, dans lequel ledit composé est le tris(8-quinolinolato)gallium(III).

14. Composition pharmaceutique comprenant : une quantité efficace d'un composé de Formule (I)

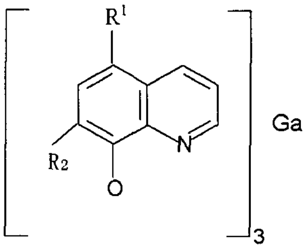

(I)

dans laquelle $R^1$ représente un atome d'hydrogène, un atome d'halogène ou un groupe sulfono $SO_3M$, dans lequel M représente un ion métal, et $R^2$ représente un atome d'hydrogène, ou $R^1$ représente un atome de Cl et $R^2$ représente un atome d'I, ou d'un sel pharmaceutiquement acceptable de celui-ci ; et une quantité efficace de témozolomide.

**15.** Composition pharmaceutique selon la revendication 14, dans laquelle ledit composé est le tris(8-quinolinolato)gallium(III).

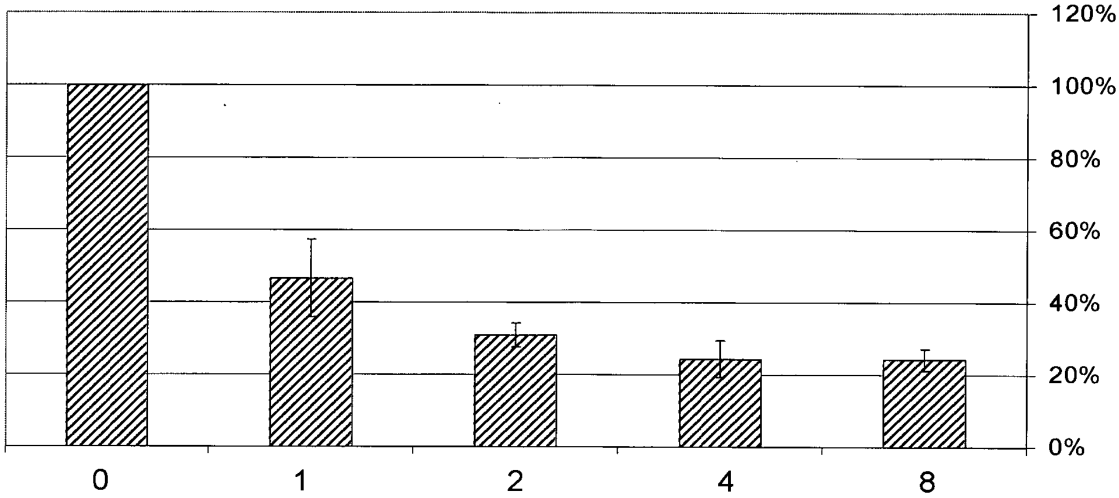


Figure 1

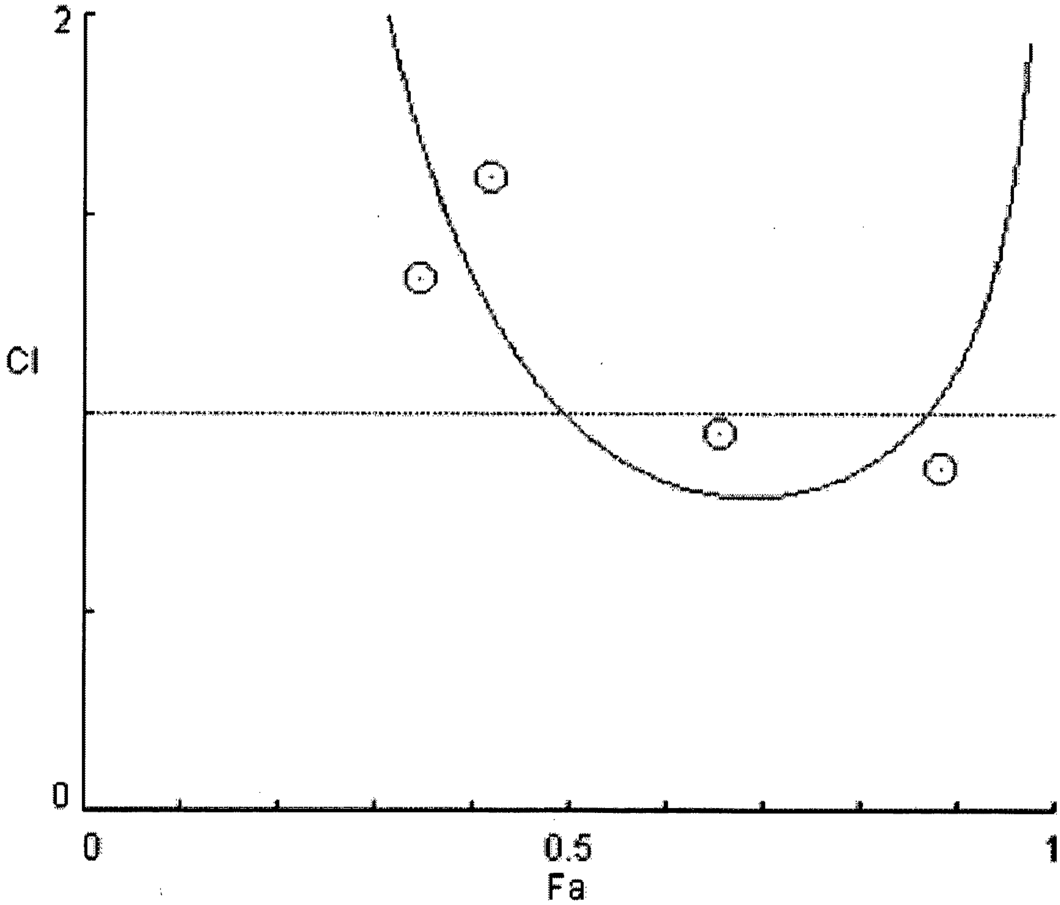


Figure 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 61308813 A
- US 61414882 A
- US 20090137620 A
- US 546506 A


### Non-patent literature cited in the description


- **SEIED MOJTABA VALIAHDI et al.** *MELANOMA RESEARCH,* 01 October 2009, vol. 19 (5), 283-293
- **THERASSE et al.** *J. Natl. Cancer Inst.,* 2000, vol. 92, 205-216
- **KANZAWA et al.** *J. Neurosurg.,* 2003, vol. 99, 1047-1052
- **BOWLES et al.** *J. Neurosurg.,* 1990, vol. 73, 248-253
- **BELJANSKI et al.** *Anticancer Res.,* 1993, vol. 13 (6A), 2301-8
- **CHOU.** *Pharmacol. Rev.,* 2006, vol. 58 (3), 621-81